# EUROPÄISCHE PATENTSCHRIFT

(11) **EP 3 052 001 B1**
(45) Veröffentlichungstag und Bekanntmachung des Hinweises auf die Patenterteilung: **22.09.2021**
(21) Anmeldenummer: 14776662.0
(22) Anmeldetag: 26.09.2014
(51) Int. Cl.: A61B 3/10, A61B 3/103

(54) **VERFAHREN ZUR BESTIMMUNG DES HORNHAUT-ASTIGMATISMUS MITTELS OPTISCHER KOHÄRENZTOMOGRAPHIE**
METHOD FOR DETECTING CORNEAL ASTIGMATISM USING OPTICAL COHERENCE TOMOGRAPHY
PROCÉDÉ DE DÉTERMINATION DE L'ASTIGMATISME DE LA CORNÉE PAR TOMOGRAPHIE PAR COHÉRENCE OPTIQUE

(30) Priorität: 30.09.2013 DE 102013219810
(43) Veröffentlichungstag der Anmeldung: 10.08.2016
(73) Patentinhaber: Carl Zeiss Meditec AG, 07745 Jena (DE)
(72) Erfinder: ANDREWS, Peter Delbert, 73447 Oberkochen (DE); RILL, Michael Stefan, 07751 Jena (DE)
(86) Internationale Anmeldenummer: PCT/EP2014/070642
(87) Internationale Veröffentlichungsnummer: WO 2015/044364

(56) Entgegenhaltungen:
- WO-A1-2010/117386
- US-A1- 2012 140 174
- MAOLONG TANG ET AL: "Corneal power measurement with Fourier-domain optical coherence tomography", JOURNAL CATARACT AND REFRACTIVE SURGERY, SURGERY, FAIRFAX, VA, US, Bd. 36, Nr. 12, 9. Juli 2010 (2010-07-09), Seiten 2115-2122, XP028170034, ISSN: 0886-3350, DOI: 10.1016/J.JCRS.2010.07.018 [gefunden am 2010-09-27]
- YOUNG HOON HWANG ET AL: "Astigmatism and optical coherence tomography measurements", GRAEFE'S ARCHIVE FOR CLINICAL AND EXPERIMENTAL OPHTHALMOLOGY, Bd. 250, Nr. 2, 1. Februar 2012 (2012-02-01), Seiten 247-254, XP055162431, ISSN: 0721-832X, DOI: 10.1007/s00417-011-1788-4

## Beschreibung

Die vorliegende Erfindung betrifft ein Verfahren zur Bestimmung refraktiver Fehler im Auge, welche auf eine suboptimale Oberflächenform der Vorderfläche und/oder Rückfläche der Hornhaut eines Auges zurückzuführen sind. Insbesondere dient das Verfahren der Bestimmung des Hornhaut-Astigmatismus mittels Optischer Kohärenztomographie.

Refraktive Fehler im Auge können unter anderem auf refraktive Fehler in der Augenlinse oder auf eine suboptimale Oberflächenform der Vorder- und Rückseite der Hornhaut zurückgeführt werden. Der refraktive Fehler der Augenlinse wird in der Regel aus dem subjektiv bestimmten Gesamtsehfehler des Auges und den refraktiven Eigenschaften der Hornhaut berechnet.

Nach dem bekannten Stand der Technik existieren unterschiedliche Verfahren, um die beiden Oberflächenformen und die Dicke der Hornhaut zu bestimmen.

Mit einem Spaltlampenmikroskop, einem der wichtigsten Untersuchungsgeräte der Augenheilkunde kann die Hornhaut in der Regel lediglich einer qualitativen Untersuchung durch einen Augenarzt oder Augenoptikermeister unterzogen werden. Der auf die Hornhaut projizierte Lichtspalt legt einen optischen Schnitt durch die Hornhaut, welcher mit verschiedenen Vergrößerungsstufen betrachtet wird. Durch verschiedene Belichtungsmethoden (diffus, direkt, fokal, indirekt, regredient, seitlich usw.) und variable Lichtspaltbreiten ist es möglich, neben den vorderen auch die mittleren und hinteren Abschnitte des Auges zu inspizieren. Eine Bestimmung der Oberflächenformen und der Dicke der Hornhaut sind jedoch nicht möglich.

Zur Bestimmung der Oberflächenformen und/oder der Dicke der Hornhaut verwenden aktuelle Systeme aufwendige Mess- und Auswerteverfahren. Einige Systeme sind dabei ausschließlich für derartige Aufgaben geeignet.

So dient beispielsweise ein Pachymeter ausschließlich der Messung der Hornhautdicke am menschlichen Auge. Die Bestimmung der Hornhautdicke ist zum einen relevant für die korrekte Bestimmung des Augeninnendruckes mittels Tonometrie. Zum anderen kommt der Pachymetrie bei der Vorbereitung auf verschiedene Augenoperationen eine weitere wichtige Rolle zu.

Dabei kommen in der Regel folgende zwei unterschiedliche Verfahren zum Einsatz:
- die kontaktfreie optische Messung (Optisches Kohärenz-Pachymeter, englisch OCP) sowie
- die Bestimmung mittels Ultraschall, bei der ein kleiner Ultraschallkopf auf die Hornhaut aufgesetzt wird.

Im Gegensatz dazu ist das Ophthalmometer (oder auch Keratometer) ein Instrument zur Messung der Oberflächenkrümmung der Hornhaut des Auges, sowie zur Bestimmung der Hornhautverläufe (Keratometrie). Das Messgerät erlaubt das Vermessen des virtuellen Bildes und somit einen Rückschluss auf die Krümmung der spiegelnden Fläche. Dabei wird ein beleuchtetes Objekt in einem bekannten Abstand aufgestellt und die Reflexion der Hornhaut beobachtet. Diese Messmethode findet gegenwärtig vorrangig in der Augenoptik bei der Anpassung von Kontaktlinsen Anwendung, wobei das Ophthalmometer vermehrt durch seine Weiterentwicklung, dem Videokeratometer ersetzt wird.

Ein weiteres, computerunterstütztes Messsystem für die exakte Vermessung der Hornhautoberfläche stellt der Keratograph dar. Hier wird die Krümmung der Hornhaut, also der Augenvorderfläche, großflächig erfasst, was etwa einer Zahl von ca. 22.000 Messpunkten entspricht. Dazu werden Testmarken in Form von Ringen auf die Hornhaut projiziert und deren Spiegelbild zur Berechnung der Hornhautkrümmung herangezogen. Aus der Abweichung dieser Ringbilder von der idealen Kreisform kann nun die Krümmungsverteilung berechnet und durch Umsetzung der Messdaten eine dreidimensionale "Landkarte" der Hornhaut bestimmt werden.

Moderne, bildgebende Hornhaut-Tomographie-Systeme basieren beispielsweise auf rotierenden Scheimpflugkameras oder scannenden Spalt-Systemen. Durch die Kombination mit Placidoscheiben kann der Bereich der Bildgebung, insbesondere der vorderen Augensegmente erheblich verbessert werden. Diese neuen Tomographen schaffen dreidimensionale Modelle der Hornhaut und ermöglichen die direkte Messung beider Hornhautoberflächen.

Die aktuell verfügbaren Systeme haben die gemeinsamen Nachteile, dass sie zum einen grundsätzlich nur den Vorderabschnitt des Auges charakterisieren können und dass sie dabei ausschließlich für derartige Aufgaben geeignet sind.

Der vorliegenden Erfindung liegt die Aufgabe zugrunde, vorhandene ophthalmologische Geräte so weiter zu entwickeln bzw. zu ergänzen, dass mit diesen neben den bisherigen Messaufgaben eine Bestimmung der auf eine suboptimale Vorder- und/oder Rückflächenform der Hornhaut zurückzuführende refraktiven Fehler im Auge möglich wird.

Diese Aufgabe wird mit dem erfindungsgemäßen Verfahren zur Bestimmung refraktiver Fehler im Auge, welche auf eine suboptimale Oberflächenform der Vorder- und/oder Rückfläche der Hornhaut zurück zu führen sind, dadurch gelöst, dass ein OCT-Volumenscan und/oder OCT-Linienscan des vorderen Augenabschnittes durchgeführt, dass aus den Messwerten durch Kantendetektion Vorder- und Rückfläche der Hornhaut erkannt und aus diesen Topographien refraktive Fehler bestimmt werden.

Erfindungsgemäß wird die Aufgabe durch die Merkmale des unabhängigen Anspruchs gelöst. Bevorzugte Weiterbildungen und Ausgestaltungen sind Gegenstand der abhängigen Ansprüche.

Das vorgeschlagene Verfahren dient zur Bestimmung refraktiver Fehler im Auge, welche auf eine suboptimale Oberflächenform der Vorder- und/oder Rückseite deren Hornhaut zurückzuführen sind. Da das erfindungsgemäße Verfahren auf OCT-Scans basiert, erweitert es damit den Anwendungsbereich reiner Standard-OCT-Systeme und integrierter OCT-Systeme. Voraussetzung dafür ist, dass die verwendeten OCT-Systeme für die Untersuchung der vorderen Augensegmente ausgelegt sind, was allerdings bei den in der Ophthalmologie verwendeten Systemen in der Regel auch der Fall ist. Weiterhin sollten die OCT-Systeme über verschiedene Scanmodi verfügen, deren Scanrichtungen individuell anpassbar sind. Die OCT-Systeme können dabei sowohl auf "Time-Domain"- als auch auf "Frequency-Domain"-Verfahren und insbesondere auch auf einem "Swept-Source"- System basieren.

Die Erfindung wird nachfolgend anhand von Ausführungsbeispielen näher beschrieben. Dazu zeigen
- Figur 1:: das OCT-Signal eines aufgefalteten Ringscans des zu untersuchenden Auges mit den durch Kantendetektion erkannten Verläufen der Hornhaut,
- Figur 2:: das zu untersuchende Auge mit eingezeichnetem Ringscan und den daraus ermittelten Meridianachsen des Auges und
- Figur 3:: die aus einem B-Scan entlang einer der Meridianachsen rekonstruierte Hornhaut mit Kreisfits an deren Vorder- und Rückseite.

Bei dem erfindungsgemäßen Verfahren zur Bestimmung refraktiver Fehler im Auge, welche auf eine suboptimale Oberflächenform der Vorder- und/oder Rückfläche der Hornhaut zurück zu führen sind, werden ein OCT-Volumenscan und/oder ein oder mehrere OCT-Linienscans des vorderen Augenabschnittes durchgeführt, aus den Messwerten durch Kantendetektion Vorder- und Rückfläche der Hornhaut detektiert, die Topographie von Vorder- und Rückfläche der Hornhaut ermittelt und aus diesen Topographien refraktive Fehler bestimmt.

Vorzugsweise kann zusätzlich zu der Topographie von Vorder- und Rückfläche die Dicke der Hornhaut ermittelt wird.

Mit Hilfe eines OCT-Scans des vorderen Augenabschnitts ist die Oberfläche und Dicke der Hornhaut an jedem beliebigen Punkt durch Bildanalyse, insbesondere durch Kantendetektion bestimmbar. Analog zu Scheimpflug- oder Placido-Ring-Systemen lassen sich daraus Oberflächen- und Krümmungsradienkarten ableiten, aus denen sich wiederum refraktive Bildfehler im Auge bestimmen lassen.

Hierbei können sowohl die als "normal" bezeichneten Abbildungsfehler (Kurzsichtigkeit, Weitsichtigkeit und Astigmatismus), als auch die sogenannten Abbildungsfehler höherer Ordnung bestimmt werden. Die "normalen Abbildungsfehler" des Auges lassen sich durch sphärische oder zylindrische Korrektur mit einer Brille ausgleichen. Im Gegensatz dazu kann man sich die Abbildungsfehler höherer Ordnung vereinfacht als winzige Unregelmäßigkeiten vorstellen, die verhindern, dass sich alle einfallenden Lichtstrahlen exakt auf der Stelle des schärfsten Sehens fokussiert werden. Eine Korrektur ist somit kaum möglich.

Gemäß einer ersten vorteilhaften Ausgestaltung des erfindungsgemäßen Verfahrens werden bei astigmatischen Augen ein OCT-Ringscan des vorderen Augenabschnittes durchgeführt und aus den Messwerten des aufgefalteten Ringscans die Extremwerte durch Kantendetektion detektiert, wobei die Minima die steile Meridianachse und die Maxima die flache Meridianachse des Astigmatismus darstellen. Dabei kann die Bestimmung der Minima und Maxima des OCT-Ringscans manuell oder automatisch erfolgen. Erfindungsgemäß erfolgt der OCT-Ringscan konzentrisch zum Augenapex.

Erfindungsgemäß ist die Charakterisierung eines astigmatischen Auges besonders einfach. Hierzu wird der Querschnitt der Hornhaut entlang eines Rings gemessen, der konzentrisch zum Augenapex liegt.

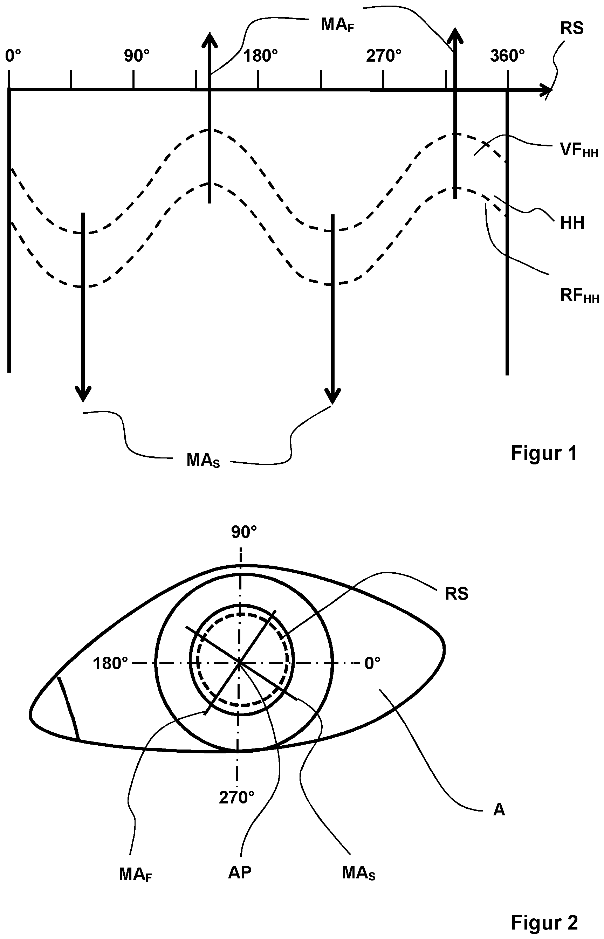

Hierzu zeigt die **Figur 1** den aufgefalteten Ringscan eines zu untersuchendes Auges mit den durch Kantendetektion detektierten Verläufen der Hornhaut. Der OCT-Ringscan (360°) wird entlang einer Linie **RS** realisiert, die konzentrisch zum Apex **AP** des Auges **A** liegt (siehe auch Figur 2). Wie der **Figur 1** zu entnehmen ist, variieren die Vorderfläche **VF_{HH}** und Rückfläche **RF_{HH}** der Hornhaut **HH** des Auges **A** nach dem "Auffalten" des OCT-Signales im Falle eines (astigmatischen) Auges **A** sinusförmig. Dabei charakterisieren die Minima die steile Meridianachse **MA_{S}** und die Maxima die flache Meridianachse **MA**_{F} des astigmatischen Auges. Somit lassen sich aus dem OCT-Ringscan die Meridianachsen des astigmatischen Auges direkt ablesen.

Die **Figur 2** zeigt das zu untersuchende Auge mit eingezeichnetem OCT-Ringscan und den daraus ermittelten Meridianachsen des astigmatischen Auges. In Übereinstimmung mit der **Figur 1** zeigt die **Figur 2** sowohl die konzentrisch zum Apex **AP** des Auges **A** liegende Linie RS des OCT-Ringscans, als auch die die beiden Meridianachsen **MA_{S}** und **MA_{F}** des astigmatischen Auges. Den Gradzahlen kann entnommen werden, wo der Beginn und das Ende des OCT-Ringscans liegen.

Gemäß einer weiteren vorteilhaften Ausgestaltung des erfindungsgemäßen Verfahrens werden entlang dieser Meridianachse B-Scans realisiert, aus den Messwerten durch Kantendetektion Vorder- und Rückfläche der Hornhaut detektiert, deren Topographie ermittelt und die Zylinderbrechkräfte bestimmt. Zur Bestimmung der Zylinderbrechkräfte werden an die Hornhautkrümmungen Kreise angefittet um die maximale und minimale Brechkraft der Hornhaut zu ermitteln.

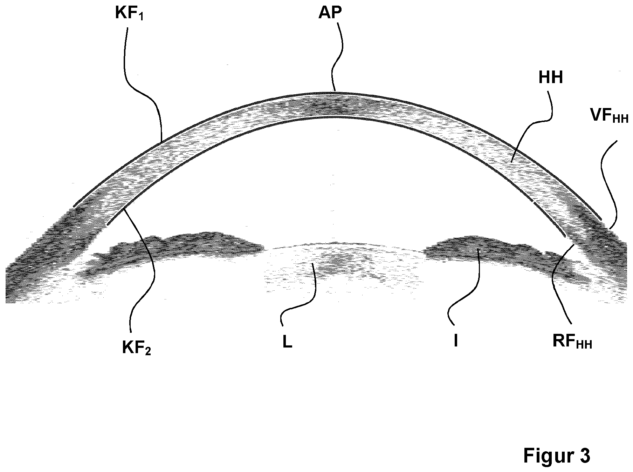

Hierzu zeigt die **Figur 3** die aus einem B-Scan entlang einer der Meridianachse rekonstruierte Hornhaut mit Kreisfits an deren Vorder- und Rückseite. Die **Figur 3** zeigt folgende, aus dem B-Scan detektierten vorderen Augensegmente: Hornhaut **HH** mit Apex **AP,** Iris **I** und Augenlinse **L**. An die Vorderfläche **VF_{HH}** und die Rückfläche **RF_{HH}** der Hornhaut **HH** sind zur Bestimmung der Zylinderbrechkräfte entsprechende Kreise **KF₁** und **KF₂** angefittet.

Gemäß einer besonders vorteilhaften Ausgestaltung des erfindungsgemäßen Verfahrens werden zur intraoperativen Bestimmung der Achslage einer implantierten torischen Intraokularlinse (kurz: IOL) aus dem aufgefalteten OCT-Ringscan des vorderen Augenabschnittes durch Kantendetektion die Extremwerte der Verläufe der Hornhaut und der implantierten torischen IOL detektiert und deren relativer Phasenunterschied berechnet.

Für den Fall, dass die aktuelle Lage der implantierten torischen IOL bekannt ist, kann die Detektion der Meridianachsen der IOL entfallen. Um die aktuelle Ausrichtung der torischen IOL einfacher erkennen zu können, ist es zweckmäßig, die Meridianachsen der IOL vor deren Implantation zu markieren.

Mit der erfindungsgemäßen Lösung wird ein Verfahren zur Verfügung gestellt, mit dem sich refraktive Fehler im Auge, welche auf eine suboptimale Oberflächenform der Vorder- und/oder Rückfläche der Hornhaut zurück zu führen sind, bestimmen lassen. Hierzu wird ein OCT-Volumenscan und/oder ein oder mehrere OCT-Linienscans des vorderen Augenabschnittes durchgeführt, aus den Messwerten durch Kantendetektion Vorder- und Rückfläche der Hornhaut detektiert, die Topographie von Vorder- und Rückfläche der Hornhaut ermittelt und aus diesen Topographien refraktive Fehler bestimmt.

Da das erfindungsgemäße Verfahren auf OCT-Scans basiert, erweitert es damit den Anwendungsbereich reiner Standard-OCT-Systeme und integrierter OCT-Systeme. Voraussetzung dafür ist, dass die verwendeten OCT-Systeme für die Untersuchung der vorderen Augensegmente ausgelegt sind. Die in der Ophthalmologie verwendeten OCT-Systeme sind in der Regel grundsätzlich in der Lage, alle optischen Komponenten des Auges in einem Messvorgang abzubilden; sei es durch Aneinanderreihen einzelner Scans oder durch Verwendung des Swept-Source-Ansatzes, der einen Gesamtaugen-Scan realisieren kann.

Weiterhin sollten die OCT-Systeme über verschiedene Scanmodi verfügen, deren Scanrichtungen individuell anpassbar sind. Die OCT-Systeme können dabei sowohl auf "Time-Domain"- als auch auf "Frequency-Domain"-Verfahren und insbesondere auch auf einem "Swept-Source"- Systems basieren.

Somit werden mit dem vorgeschlagene Verfahren ophthalmologische Geräte so weiterentwickelt bzw. ergänzt, dass mit diesen neben den bisherigen Messaufgaben eine Bestimmung der auf eine suboptimale Vorder- und/oder Rückflächenform der Hornhaut zurückzuführende refraktiven Fehler im Auge möglich wird.

Die erfindungsgemäße Lösung stellt eine schnelle und einfache Methode zur Bestimmung refraktiver Fehler im Auge mittels OCT dar. Zusammen mit einer OCT-Pachymetrie-Messungen (Bestimmung der Hornhautdicke) kann die Methode für eine Astigmatismus-Korrektur durch Einschnitte nahe des Augenlimbus benutzt werden, z. B. limbale Relaxationsschnitte mittels fs-Laser.

Ein besonderer Vorteil des vorgeschlagenen Verfahrens zur Bestimmung refraktiver Fehler im Auge, welche auf eine suboptimale Oberflächenform der Vorder- und/oder Rückfläche der Hornhaut zurück zu führen sind, ist darin zu sehen, dass sich aus dem OCT-Ringscan die Meridianachsen eines astigmatischen Auges direkt ablesen lassen.

## Patentansprüche

1. Verfahren zur Bestimmung refraktiver Fehler im Auge, welche auf eine suboptimale Oberflächenform der Vorder- und/oder Rückfläche der Hornhaut eines astigmatischen Auges zurück zu führen sind, bei dem ein OCT-Volumen-scan und/oder ein oder mehrere OCT-Linienscans des vorderen Augenabschnittes durchgeführt, aus den Messwerten durch Kantendetektion Vorder- und Rückfläche der Hornhaut detektiert, die Topographie von Vorder- und Rückfläche sowie die Dicke der Hornhaut ermittelt und aus diesen Topographien refraktive Fehler bestimmt werden, wobei ein OCT-Ringscan des vorderen Augenabschnittes durchgeführt wird, **dadurch gekennzeichnet dass** aus den Messwerten des aufgefalteten Ringscans, der entlang einer Linie (RS), die konzentrisch zum Apex (AP) des Auges (A) liegt, realisiert wird, durch Kantendetektion die Extremwerte des Verlaufes der Hornhaut detektiert werden.

2. Verfahren nach Anspruch 1, **dadurch gekennzeichnet, dass** die Bestimmung der Minima und Maxima des OCT-Ringscans manuell oder automatisch erfolgt, wobei die Minima die steile Meridianachse und die Maxima die flache Meridianachse des Astigmatismus darstellen.

3. Verfahren nach Anspruch 1, **dadurch gekennzeichnet, dass** der OCT-Ringscan konzentrisch zum Augenapex erfolgt.

4. Verfahren nach den Ansprüchen 1 und 2, **dadurch gekennzeichnet, dass** entlang dieser Meridianachsen B-Scans realisiert, aus den Messwerten durch Kantendetektion Vorder- und Rückfläche der Hornhaut detektiert, deren Topographie ermittelt und die Zylinderbrechkräfte bestimmt werden.

5. Verfahren nach Anspruch 4, **dadurch gekennzeichnet, dass** zur Bestimmung der Zylinderbrechkräfte an die Hornhautkrümmungen Kreise angefittet werden um die maximale und minimale Brechkraft der Hornhaut zu ermitteln.

6. Verfahren nach Anspruch 1, **dadurch gekennzeichnet, dass** zur intraoperativen Bestimmung der Achslage einer implantierten torischen IOL aus dem aufgefalteten OCT-Ringscan des vorderen Augenabschnittes durch Kantendetektion die Extremwerte der Verläufe der Hornhaut und der implantierten torischen IOL detektiert und deren relativer Phasenunterschied berechnet werden.

7. Verfahren nach Anspruch 6, **dadurch gekennzeichnet, dass** die Detektion der Meridianachsen der implantierten torischen IOL entfallen kann, wenn deren aktuelle Lage bekannt ist.

8. Verfahren nach Anspruch 7, **dadurch gekennzeichnet, dass** die Meridianachsen der torischen IOL vor dem Implantieren entsprechend markiert werden, um deren aktuelle Lage zu erkennen.

## Claims

1. A method for determining refractive errors in the eye which can be traced back to a sub-optimal surface shape of the front and/or back surface of the cornea of an astigmatic eye, in which method an OCT volume scan and/or one or more OCT line scans of the anterior eye portion are carried out, the front and back surfaces of the cornea are detected from the measurement values by edge detection, the topography of the front and back surfaces and the thickness of the cornea are ascertained and refractive errors are determined from these topographies, wherein an OCT ring scan of the anterior eye portion is carried out, **characterized in that** the extremal values of the curve of the cornea are detected from the measurement values of the deconvolved ring scan, which is realized along a line (RS) which is concentric to the apex (AP) of the eye (A) .

2. Method according to Claim 1, **characterized in that** the minima and maxima of the OCT ring scan are determined manually or automatically, wherein the minima represent the steep meridian axis and the maxima represent the flat meridian axis of the astigmatism.

3. Method according to Claim 1, **characterized in that** the OCT ring scan is implemented in concentric fashion with respect to the apex of the eye.

4. Method according to Claims 1 and 2, **characterized in that** B-scans are realized along these meridian axes, the front and back surfaces of the cornea are detected from the measurement values by edge detection, the topography thereof is ascertained and the cylinder refractive powers are determined.

5. Method according to Claim 4, **characterized in that**, for the purposes of determining the cylinder refractive powers, circles are fitted to the corneal curvatures in order to ascertain the maximum and minimum refractive power of the cornea.

6. Method according to Claim 1, **characterized in that**, to determine the axial position of an implanted toric IOL intraoperatively, the extremal values of the curves of the cornea and of the implanted toric IOL are detected by edge detection from the deconvolved OCT ring scan of the anterior eye portion and the relative phase difference thereof is calculated.

7. Method according to Claim 6, **characterized in that** the detection of the meridian axes of the implanted toric IOL can be dispensed with if the current position thereof is known.

8. Method according to Claim 7, **characterized in that** the meridian axes of the toric IOL are marked appropriately prior to implantation in order to identify the current position thereof.

## Revendications

1. Procédé pour déterminer des défauts de réfraction dans l'œil, lesquels sont à attribuer à une forme de surface suboptimale de la surface avant et/ou arrière de la cornée d'un œil astigmatique, avec lequel un balayage de volume par tomographie en cohérence optique et/ou un ou plusieurs balayages linéaires par tomographie en cohérence optique de la portion d'œil avant sont effectués, les surfaces avant et arrière de la cornée sont détectées à partir des valeurs mesurées par détection d'arête, la topographie des surfaces avant et arrière ainsi que l'épaisseur de la cornée sont identifiées et des défauts de réfraction sont déterminés à partir de ces topographies, un balayage annulaire de tomographie en cohérence optique de la portion avant de l'œil étant effectué, **caractérisé en ce que** les valeurs extrêmes du tracé de la cornée sont détectées par détection des arêtes à partir des valeurs mesurées du balayage annulaire déplié, lequel est réalisé le long d'une ligne (RS) qui est concentrique au sommet (AP) de l'œil (A).

2. Procédé selon la revendication 1, **caractérisé en ce que** la détermination du minimum et du maximum du balayage annulaire de tomographie en cohérence optique est effectuée manuellement ou automatiquement, le minimum représentant l'axe méridien raide et le maximum l'axe méridien plat de l'astigmatisme.

3. Procédé selon la revendication 1, **caractérisé en ce que** le balayage annulaire de tomographie en cohérence optique est effectué de manière concentrique par rapport au sommet de l'œil.

4. Procédé selon les revendications 1 et 2, **caractérisé en ce que** des balayages B sont réalisés le long de ces axes méridiens, les surfaces avant et arrière de la cornée sont détectées à partir des valeurs mesurées par détection d'arête, leur topographie est identifiée et les pouvoirs réfringents de cylindre sont déterminés.

5. Procédé selon la revendication 4, **caractérisé en ce qu'**en vue de déterminer les pouvoirs réfringents de cylindre, des cercles sont ajustés sur les courbures de la cornée afin d'identifier le pouvoir réfringent minimal et maximal de la cornée.

6. Procédé selon la revendication 1, **caractérisé en ce qu'**en vue de la détermination peropératoire de la position d'axe d'une IOL torique implantée, les valeurs extrêmes des tracés de la cornée et de l'IOL torique implantée sont détectées par détection des arêtes à partir du balayage annulaire de tomographie en cohérence optique déplié de la portion avant de l'œil et leur différence de phases relative est calculée.

7. Procédé selon la revendication 6, **caractérisé en ce que** la détection des axes méridiens de l'IOL torique implantée peut être ignorée lorsque leur position actuelle est connue.

8. Procédé selon la revendication 7, **caractérisé en ce que** les axes méridiens de l'IOL torique sont marqués en conséquence avant l'implantation afin de reconnaître leur position actuelle.
